# FASCICULE DE BREVET EUROPEEN

(11) **EP 4 651 801 B1**
(45) Date de publication et mention de la délivrance du brevet: **09.12.2026**
(21) Numéro de dépôt: 24703602.3
(22) Date de dépôt: 12.01.2024
(51) Int. Cl.: A61B 5/349

(54) **DÉTERMINATION D'UN RISQUE DE DÉCOMPENSATION CARDIAQUE D'UN PATIENT**
BESTIMMUNG EINES RISIKOS EINER HERZDEKOMPENSATION EINES PATIENTEN
DETERMINING A RISK OF CARDIAC DECOMPENSATION OF A PATIENT

(30) Priorité: 18.01.2023 FR 2300451
(43) Date de publication de la demande: 26.11.2025
(73) Titulaire: Cardiametrics, 38240 Meylan (FR)
(72) Inventeur: MICHEL, Cindy, 38700 La Tronche (FR); TUYISENGE, Viateur, 38100 Grenoble (FR)
(74) Mandataire: Ex Materia
(86) Numéro de dépôt international: PCT/FR2024/050045
(87) Numéro de publication internationale: WO 2024/153879

(56) Documents cités:
- WO-A1-2015/065674
- WO-A1-2021/019027
- US-A1- 2013 116 578

## Description

La présente invention concerne le domaine des dispositifs médicaux et des systèmes de surveillance de la santé cardiaque d'un être vivant. La présente invention concerne plus particulièrement un système de détermination d'une décompensation d'une insuffisance cardiaque au sein duquel un dispositif médical implantable et communiquant avec un serveur informatique est destiné à mesurer des paramètres cardiaques. L'art antérieur pertinent est décrit dans WO 2021/019027 A1, WO 2015/065674 A1, US 2013/116578 A1.

L'insuffisance cardiaque est une affection chronique touchant une large partie de la population, notamment les plus de 60 ans, et cette affection peut notamment générer des épisodes de décompensation dont la fréquence augmente avec l'altération de la fonction cardiaque.

Les épisodes de décompensation de l'insuffisance cardiaque sont souvent accompagnés d'une hospitalisation en urgence du patient souffrant de cette affection cardiaque du fait qu'ils ne sont pas détectés suffisamment en amont, l'épisode de décompensation de l'insuffisance cardiaque apparaissant régulièrement comme asymptomatique à ses débuts. Cette absence de symptômes détectables par le patient implique que l'épisode de décompensation de l'insuffisance cardiaque évolue sans qu'une action préventive ne soit effectuée. Or, dès lors que le patient commence à sentir les premiers symptômes de la décompensation de l'insuffisance cardiaque, comme une fatigue, des palpitations ou encore un essoufflement, l'hospitalisation est difficile à éviter.

On comprend de ce qui précède que plus la détection de l'épisode de décompensation de l'insuffisance cardiaque survient tôt et moins le risque d'hospitalisation, et les coûts qu'elle peut générer, est grand, la détection précoce d'une décompensation d'une insuffisance cardiaque chez le patient permettant d'intervenir sur ce dernier en lui prescrivant un traitement médicamenteux permettant de stabiliser la fonction cardiaque du patient.

La détection précoce d'une décompensation d'une insuffisance cardiaque peut notamment reposer sur une analyse approfondie de différents paramètres cardiaques infracliniques, et plus particulièrement de paramètres hémodynamiques.

La présente invention s'inscrit dans ce contexte et se propose de fournir un système de détermination d'un épisode de décompensation de l'insuffisance cardiaque chez un patient.

Ainsi, la présente demande divulgue un procédé de détermination d'un risque de décompensation cardiaque d'un patient au cours duquel on réalise une étape de traitement d'un signal accélérométrique acquis sur le patient et une étape de détermination d'un risque de décompensation cardiaque basée sur l'étude d'une pluralité de marqueurs parmi lesquels des segments d'un signal accélérométrique traité résultant de ladite étape de traitement,
le procédé étant d'une part caractérisé en ce que l'étape de traitement du signal accélérométrique comporte :
   - une étape de découpage du signal accélérométrique en cycles temporels d'une même durée,
   - une étape d'identification, dans chaque cycle temporel, de segments caractéristiques du son cardiaque, avec une première recherche d'un premier type de segment dans une première partie définie du cycle temporel et une deuxième recherche d'au moins un deuxième type de segment et un troisième type de segment dans une deuxième partie définie du cycle temporel,
   - une étape de sélection de la cohérence, au moins pour les premiers types de segments et les deuxièmes types de segments, des segments identifiés,
   - une étape de calcul d'un segment d'un premier type moyen, d'un segment d'un deuxième type moyen et d'un segment d'un troisième type moyen, le calcul de moyenne étant basé sur tous les segments d'un premier type sélectionnés, respectivement tous les segments d'un deuxième type sélectionnés, respectivement tous les segments d'un troisième type sélectionnés,
le procédé étant d'autre part caractérisé en ce que l'étape de détermination comporte au moins une étape de détermination d'une augmentation de l'amplitude du premier type de segment par rapport à des données en mémoire et/ou d'une diminution de l'amplitude du troisième type de segment par rapport à des données en mémoire.

Le procédé est ainsi particulier en ce qu'il permet de déterminer un risque de décompensation sur la base d'un son cardiaque représentatif d'une période d'acquisition donnée, ce son cardiaque représentatif résultant d'une compilation de sons cardiaques obtenu par traitement d'au moins un signal accélérométrique.

Le signal accélérométrique est découpé en une pluralité de cycles temporels de même durée. Par même durée, il convient de comprendre que la durée des cycles temporels d'un signal accélérométrique acquis peut être différente de la durée des cycles temporels d'un autre signal accélérométrique précédemment acquis sur le même patient, mais que la durée des cycles temporels d'un même signal accélérométrique est la même pour chaque cycle temporel à la fin de l'étape de découpage.

La durée peut être définie préalablement à la mise en œuvre du procédé ou bien elle peut être définie en cours de procédé, par le calcul, en fonction des caractéristiques des cycles temporels propres au signal accélérométrique en cours d'analyse. Dans un exemple non limitatif de l'invention, on hache dans un premier temps le signal accélérométrique en cycles temporels, en utilisant un paramètre d'identification pour débuter le découpage de la période et un autre paramètre d'identification, ou le même, pour finir le découpage de la période, de sorte que chaque cycle a une durée qui lui est propre, reflétant l'apparition réelle de ces paramètres d'identification. On fait ensuite la moyenne des durées des cycles temporels, pour déterminer la durée à donner aux cycles temporels pour la suite du procédé selon l'invention. Et on ajuste la durée de chaque cycle temporel résultant du hachage initial pour lui donner une durée égale à cette durée moyenne. Selon la durée initiale du cycle temporel, l'unité de commande peut couper le cycle temporel pour réduire sa durée et lui donner une durée égale à la durée moyenne ou bien allonger le cycle temporel pour augmenter sa durée. Dans ce dernier cas, l'unité de commande est configurée pour ajouter une ou plusieurs valeurs de signal nulles en fin de cycle temporel, les valeurs étant ajoutées à intervalles réguliers en fonction de la fréquence d'échantillonnage. A titre d'exemple, pour un échantillonnage de 500 Hz, une valeur de signal nulle est ajoutée toutes les deux millisecondes en fin de cycle temporel.

Pour chaque cycle temporel défini dans la période d'acquisition donnée, différents segments du son cardiaque sont identifiés et le traitement du signal accélérométrique se fait avantageusement par type de segments, avec notamment les segments de premier type d'un côté, et les segments de deuxième type et de troisième type d'un autre côté, ces deux derniers types de segments étant traités ensembles ou parallèlement. Ceci présente l'avantage de pouvoir récupérer une information valable sur un segment d'un type pour un cycle temporel donné alors que l'information relative au segment d'un autre type est à exclure pour le même cycle temporel, ce qui offre la possibilité d'augmenter le nombre de segments d'un même type à compiler pour une période d'acquisition. En d'autres termes, pour un cycle temporel donné, s'il n'est pas identifié de segment de premier type ou que le segment de premier type identifié semble être trop différent de ce qui est constaté dans les autres cycles temporels, notamment du fait d'un problème d'acquisition ponctuel, il est possible de récupérer une information utilisable pour le segment de deuxième type et/ou pour le segment de troisième type.

Selon une caractéristique optionnelle, l'étape d'identification comporte une sous-étape d'exclusion de segments préalablement identifiés comme étant des segments de premier, deuxième ou troisième types, et classés lors de cette sous-étape d'exclusion comme étant des artefacts.

De tels artefacts peuvent notamment apparaître lorsque l'acquisition du signal se fait simultanément à un mouvement du patient pendant son sommeil, et/ou simultanément à un ronflement ou à un épisode de toux, sans que ces exemples soient limitatifs de l'invention. On comprend que les segments identifiés comme des artefacts, c'est-à-dire des segments qui sont trop éloignés de la forme d'un segment physiologique, que ce soit en durée ou en amplitude, sont exclus de l'étape de sélection de la cohérence, ce qui permet d'éviter d'engager du temps de calcul par une unité de commande pour des segments qui ne seront de toute façon pas gardés à la suite de cette étape de sélection et qui pourraient altérer l'analyse. On améliore la rapidité de traitement de l'information et on évite que l'étape de sélection de cohérence soit polluée par des segments non caractéristiques de l'état du patient.

Selon une caractéristique optionnelle, la sous-étape d'exclusion comporte une opération de calcul d'un score d'analyse spectrale, les segments présentant un score supérieur à une valeur seuil étant identifiés comme étant des artefacts et exclus. Notamment, le score d'analyse spectrale est un z-score, et la valeur seuil est égale à 3.

Selon une caractéristique optionnelle, les segments de troisième type sont détectés dans la deuxième partie du cycle temporel à la suite des segments de deuxième type détectés.

Selon une caractéristique optionnelle, les segments de troisième type ont une amplitude moyenne plus petite que l'amplitude moyenne des segments de deuxième type.

Selon une caractéristique optionnelle, le procédé de détermination comporte une étape d'acquisition d'au moins un signal accélérométrique du patient, préalablement à l'étape de traitement.

Selon une caractéristique optionnelle, l'étape d'acquisition est réalisée par un accéléromètre trois axes, l'étape de traitement étant réalisée pour chacun des signaux détectés sur l'un des trois axes ou bien pour un signal d'ensemble résultant de la combinaison des signaux acquis sur chacun des axes.

Selon une caractéristique optionnelle, l'étape d'acquisition d'au moins un signal accélérométrique est accompagnée d'une étape acquisition d'un signal électrocardiogramme.

Selon une caractéristique optionnelle, l'étape de découpage du signal comporte une opération de synchronisation du signal accélérométrique sur la base du signal électrocardiogramme.

Selon une caractéristique optionnelle, l'étape d'identification comporte une étape de calcul d'enveloppe du signal, un segment étant identifié lorsque l'amplitude de l'enveloppe est supérieure à un seuil déterminé dans une partie définie du cycle temporel.

Selon une caractéristique optionnelle, le calcul d'enveloppe du signal est réalisé sur l'intégralité du cycle.

Selon une caractéristique optionnelle, le seuil déterminé est de l'ordre de 10% de la valeur maximale d'amplitude.

Selon une caractéristique optionnelle, l'étape de calcul d'enveloppe comporte aussi bien un calcul d'enveloppe au carré qu'un calcul de valeur absolue.

Selon une caractéristique optionnelle, la première partie définie du cycle temporel dans l'étape de recherche est égale au premier tiers de la durée dudit cycle temporel, la deuxième partie définie du cycle temporel étant égale au deux derniers tiers dudit cycle temporel.

Selon une caractéristique optionnelle, l'étape de sélection comporte au moins deux suites de calculs de corrélation effectuées en parallèle, parmi lesquels une première suite de calculs de corrélation réalisée sur l'ensemble des segments du premier type et une deuxième suite de calculs de corrélation réalisée sur l'ensemble des segments du deuxième type.

Selon une caractéristique optionnelle, chaque suite de calculs de corrélation effectuée pour un type de segment donné comporte un premier calcul d'intercorrélation entre l'ensemble des segments identifiés comme étant des segments de ce type, pour définir un segment de référence pour ce type de segment, puis des étapes de calcul de corrélation de chacun des segments identifiés comme étant des segments de ce type par rapport au segment de référence.

Selon une caractéristique optionnelle, les segments sélectionnés dans chaque suite de calculs de corrélation sont les segments qui ont un indice de corrélation avec le segment de référence qui est supérieur ou égal à un seuil de corrélation prédéterminé. A titre d'exemple, le seuil de corrélation est au moins égal à 50%, et par exemple de l'ordre de 60%. En d'autres termes, tous les segments qui présentent un indice de corrélation inférieur au seuil de corrélation, 60% dans ce dernier exemple, sont mis de côté et ne sont pas considéré pour l'étape de calcul d'un segment moyen.

Selon une caractéristique optionnelle, l'étape de sélection comporte uniquement deux suites de calculs de corrélation effectuées en parallèle, parmi lesquels une première suite de calcul de corrélation réalisée sur l'ensemble des segments du premier type et une deuxième suite de calcul de corrélation réalisée sur l'ensemble des segments du deuxième type.

Dans cette variante, aucun calcul d'intercorrélation n'est effectué pour les segments de troisième type alors qu'ils ont été identifiés préalablement. Dans ce cas, le tri par cohérence par rapport à un segment de référence n'est effectué que via les segments de deuxième type. Le segment de troisième type est trié de la même façon que le segment de deuxième type présent dans le même cycle temporel : si un segment de deuxième type est conservé après l'étape de tri par cohérence, le segment de troisième type du même cycle temporel est conservé, tandis que si un segment de deuxième type est exclu après l'étape de tri par cohérence, le segment de troisième type du même cycle temporel est également exclu.

En d'autres termes, on fait un traitement de signal qui vise à considérer le segment de troisième type car c'est l'un des plus spécifiques pour la détermination d'une insuffisance cardiaque, mais ce traitement de signal est spécifique en ce que le segment moyen de troisième type est calculé sur la base de segments de troisième type qui ont été exclus ou conservés en fonction de l'exclusion ou de la conservation des segments du deuxième type du même cycle temporel, l'identification de ces segments de deuxième type étant plus certaine.

On segmente un signal accélérométrique en trois segments et l'on traite en deux procédures distinctes les premiers segments d'une part et les deuxièmes et troisièmes segments d'autre part, le traitement des deuxièmes et troisièmes segments se faisant par une intercorrélation et une sélection basée sur les seuls deuxièmes segments.

Selon une caractéristique optionnelle, l'étape de sélection comporte trois suites de calculs de corrélation effectuées en parallèle, parmi lesquels une première suite de calcul de corrélation réalisée sur l'ensemble des segments du premier type S1, une deuxième suite de calcul de corrélation réalisée sur l'ensemble des segments du deuxième type S2, et une troisième suite de calcul de corrélation réalisée sur l'ensemble des segments du troisième type. En d'autres termes, des calculs d'intercorrélation sont effectués pour chacun des segments identifiés préalablement.

Selon une caractéristique optionnelle, on effectue, entre l'étape de sélection et l'étape de calcul des segments moyens, une étape de recalage temporel des segments sélectionnés sur le segment de référence correspondant, pour chaque type de segments.

Selon une caractéristique optionnelle, l'étape de détermination comporte en outre une étape de détermination d'une augmentation de l'amplitude du deuxième type de segment par rapport à des données en mémoire. Une telle augmentation peut notamment être le signe d'hypertension artérielle pulmonaire.

D'autres caractéristiques, détails et avantages de l'invention ressortiront plus clairement à la lecture de la description qui suit d'une part, et d'exemples de réalisation donnés à titre indicatif et non limitatif en référence aux dessins schématiques annexés d'autre part, sur lesquels :
[Fig.1] représente les principales étapes d'un procédé de détermination d'une décompensation cardiaque ;
[Fig.2] représente un premier mode de réalisation du procédé illustré sur la figure 1 ;
[Fig.3] représente un signal accélérométrique découpé en cycles temporels au cours d'une des étapes du procédé ;
[Fig.4] représente le signal accélérométrique de la figure 3, avec un calcul d'enveloppe au carré et d'enveloppe en valeur absolue permettant la mise en œuvre d'étapes ultérieures du procédé ;
[Fig.5] représente un cycle temporel moyen représentatif du signal accélérométrique traité, avec d'une part un segment d'un premier type moyen dans un premier tiers du cycle et d'autre part un segment d'un deuxième type moyen et un segment d'un troisième type moyen dans les autres deux tiers ;
[Fig.6] représente un deuxième mode de réalisation du procédé illustré sur la figure 1.

Les caractéristiques, les variantes et les différentes formes de réalisation de l'invention peuvent être associées les unes avec les autres, selon diverses combinaisons, dans la mesure où elles ne sont pas incompatibles ou exclusives les unes des autres. On pourra notamment imaginer des variantes de l'invention ne comprenant qu'une sélection de caractéristiques décrites par la suite de manière isolées des autres caractéristiques décrites, si cette sélection de caractéristiques est suffisante pour conférer un avantage technique.

Sur les figures, les éléments communs à plusieurs figures conservent la même référence.

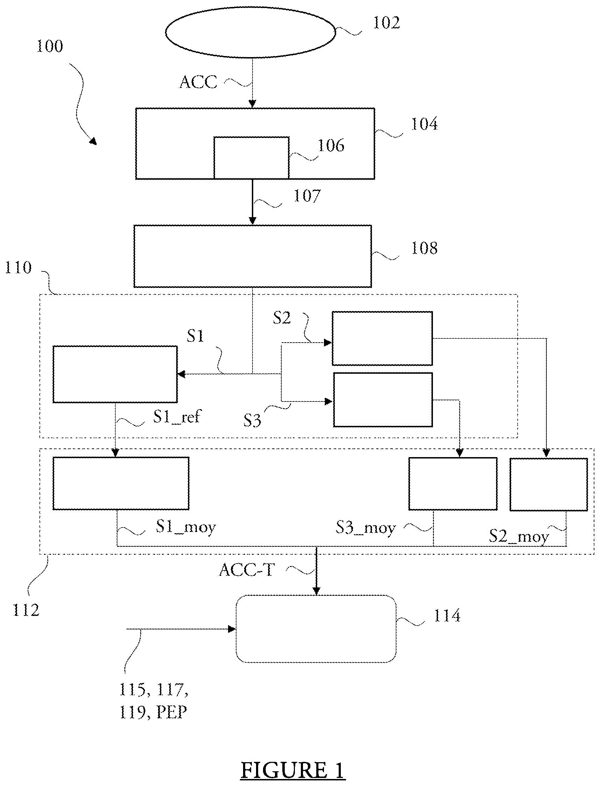

La figure 1 illustre un procédé de détermination 100 d'un risque de décompensation cardiaque d'un patient, au cours duquel on réalise principalement une étape de traitement d'un signal accélérométrique acquis sur le patient et une étape de détermination d'un risque de décompensation cardiaque basée sur l'étude d'une pluralité de marqueurs parmi lesquels ledit signal accélérométrique traité.

La figure 1 illustre plus particulièrement les grandes lignes du procédé de détermination 100 avec une étape d'acquisition 102 d'au moins un signal accélérométrique ACC, l'étape de traitement 104 du signal accélérométrique qui comporte au moins une étape de découpage 106 du signal accélérométrique en cycles temporels 107 d'une durée déterminée, une étape d'identification 108, dans chaque cycle temporel 107, de segments S1, S2, S3 caractéristiques du son cardiaque, une étape de sélection 110 de la cohérence des segments identifiés, une étape de calcul 112 de segments moyens représentatifs du signal accélérométrique traité ACC-T, et l'étape de détermination d'un risque de décompensation cardiaque 114.

L'étape d'acquisition 102 d'au moins un signal accélérométrique ACC peut être réalisée par tout moyen d'acquisition possible, dès lors que le moyen d'acquisition choisi permet l'acquisition d'un signal le plus représentatif possible de l'activité cardiaque, avec le moins possible d'artefact. A titre d'exemple, pour acquérir un tel signal, il peut être judicieux de prévoir un implant sous-cutané au niveau de la cage thoracique et de planifier l'acquisition pendant le sommeil du patient, afin de s'assurer que l'acquisition est réalisée lorsque le patient ne réalise pas de mouvements parasites. Une telle stabilité physiologique permet d'assurer l'acquisition de données répétables, de sorte que les variations observées après traitement du signal accélérométrique peuvent être considérées comme dues à la pathologie.

L'accéléromètre utilisé peut être un accéléromètre trois axes, qui permet pour une période donnée d'acquérir le même signal accélérométrique représentatif de l'activité cardiaque selon trois axes distincts. Dans la description qui va suivre, le traitement d'un signal accélérométrique ACC peut être le traitement d'un signal récupéré sur un seul axe, du fait d'une sélection parmi les trois axes ou bien de l'acquisition par un accéléromètre apte à acquérir un signal selon un seul axe. Mais il convient de noter que la description s'appliquerait de manière identique si le signal accélérométrique traité était un signal résultant d'une combinaison des valeurs acquises sur chacun des trois axes.

Le moyen d'acquisition utilisé est configuré pour envoyer les données acquises à une unité de commande, qui peut être implanté sur un serveur à distance, sur un appareil mobile du patient, ou encore sur un matériel informatique connecté au moyen d'acquisition sans que cela soit limitatif de l'invention, les données pouvant être transmises par un protocole de communication sans fil, ou par des moyens filaires si le type de moyen d'acquisition mis en œuvre et la localisation de l'unité de commande le permet. L'unité de commande est configurée pour mettre en œuvre l'étape de traitement 104 du signal accélérométrique ACC.

Plus particulièrement, l'étape de découpage 106 du signal accélérométrique consiste à évaluer le signal accélérométrique ACC acquis sur l'ensemble de la période d'acquisition, qui peut être de l'ordre de 30 secondes par exemple, et à le découper en cycles temporels 107 d'une même durée. Les cycles temporels sont de même durée, dans la mesure où cette durée est la même pour chaque cycle temporel résultant du découpage d'un même signal accélérométrique et qui est ensuite analysé dans la suite du procédé.

Des moyens sont décrits dans le reste de la description pour générer des cycles temporels de même durée, et à titre d'exemple, la durée des cycles temporels d'un même signal accélérométrique peut être de l'ordre de 300 à 1500 millisecondes.

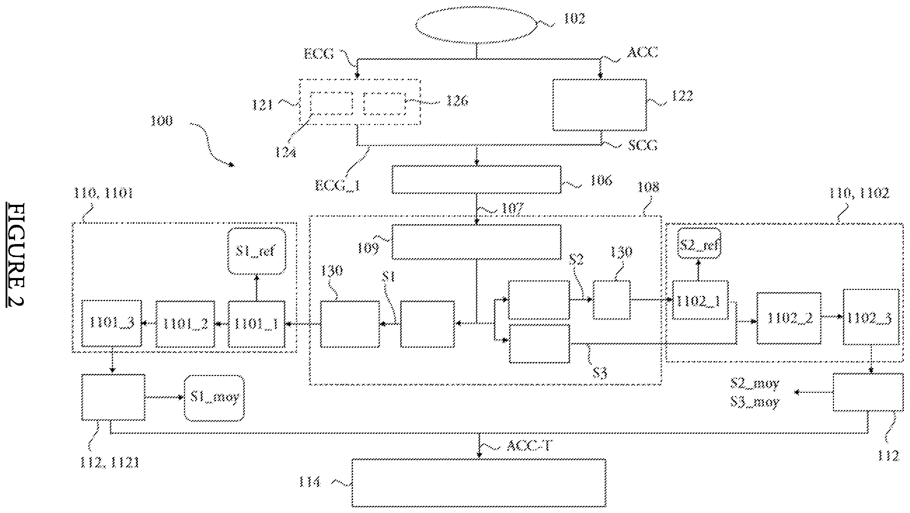

Tel que cela sera décrit ci-après, notamment en référence à la figure 2, cette étape de découpage 106 du signal accélérométrique peut être avantageusement associée au traitement simultané du signal accélérométrique ACC et d'un signal électrocardiogramme ECG acquis de manière simultanée avec ledit signal accélérométrique, de sorte que la caractéristique particulière de l'activité cardiaque, servant à donner au moins une origine aux cycles temporels 107 du signal accélérométrique ACC et le cas échéant la fin des cycles temporels, peut être détectée par analyse du signal électrocardiogramme ECG et peut par exemple correspondre à l'apparition d'un pic R.

Une fois le signal découpé en différents cycles temporels 107, au moins certains de ces cycles temporels sont analysés pour identifier, au cours de l'étape d'identification 108, des segments S1, S2, S3 caractéristiques du son cardiaque. Ces segments caractéristiques sont notamment identifiés lorsque l'amplitude du signal accélérométrique, et plus particulièrement l'amplitude d'une enveloppe du signal accélérométrique obtenue par une opération de redressement du signal, est supérieure à une valeur définie, par exemple supérieure à un pourcentage d'une valeur maximale d'amplitude de l'enveloppe constatée sur la durée du cycle temporel 107.

L'étape d'identification 108 permet notamment d'identifier au moins un premier type de segment S1 dans une première partie définie d'un des cycles temporels 107 analysés et au moins un deuxième type de segment S2 et un troisième type de segment S3 dans une deuxième partie définie de ce même cycle temporel 107. Ainsi, pour une période d'acquisition donnée, l'unité de commande est configurée pour stocker d'une part l'ensemble des segments de premier type S1 identifiés sur l'ensemble des cycles temporels 107 dans une base de données appropriée, et pour stocker de manière distinctive dans une base de données appropriée les segments S2, S3 identifiés dans les deuxièmes parties de chaque cycle temporel analysé 107.

Selon les modes de réalisation qui vont être décrit ci-après, les segments de deuxième type S2 et les segments de troisième type S3 peuvent être ensuite traités simultanément, en faisant porter le traitement uniquement sur les segments de deuxième type S2, ou bien ils peuvent être traités parallèlement, le traitement portant alors aussi bien sur les segments de deuxième type S2 que les segments de troisième type S3.

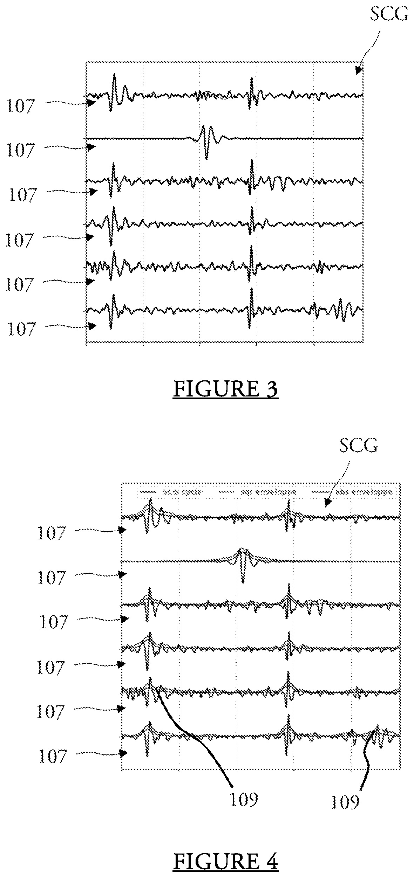

L'étape suivante est donc réalisée au moins pour les premiers types de segments S1 et pour les deuxièmes types de segments S2 et/ou les troisièmes types de segments S3, en référence à ce qui a été évoqué ci-dessus, et elle consiste en une étape de sélection 110 de la cohérence des segments identifiés. Cette étape va être décrite plus en détails ci-après, en référence aux figures 2 et 3 notamment : elle consiste notamment à intercorréler l'ensemble des segments du même type identifiés au préalable pour pouvoir en déduire un segment de référence pour ce type de segments et pour pouvoir dans un deuxième temps ne garder que les segments se rapprochant au plus près de ce segment de référence, c'est-à-dire les segments cohérents de chacun des types de segment.

Le traitement du signal se finit par une étape de calcul 112 d'un segment moyen pour chacun des types de segment, et ce quel que soit le nombre de types de segments qui ont été traités simultanément lors de l'étape précédente. En d'autres termes, que les segments du troisième type aient été traités distinctement des segments du deuxième type ou bien en ayant été rattachés à ces derniers, l'étape de calcul permet d'obtenir un segment d'un premier type moyen S1_moy, un segment d'un deuxième type moyen S2_moy et un segment d'un troisième type moyen S3_moy.

Le signal accélérométrique traité, résultant de l'étape de traitement du signal et reflétant l'activité cardiaque du patient sur la période d'acquisition du signal, ici de l'ordre de 30 secondes, est formé par la juxtaposition de chacun des segments moyens S1_moy, S2_moy, S3_moy précédemment calculés.

Tel qu'évoqué, le signal accélérométrique traité est un marqueur utilisé lors de l'étape de détermination d'un risque de décompensation cardiaque 114. Cette étape de détermination peut notamment considérer d'autres marqueurs, parmi lesquels des marqueurs électrophysiologiques 115 déterminés via le signal électrocardiogramme évoqué précédemment, la fréquence respiratoire 117, l'inclinaison du corps du patient 119 pendant la nuit ou encore la période de pré-éjection, connu sous l'acronyme PEP.

Le marqueur correspondant au signal accélérométrique traité peut notamment être utilisé pour réaliser une comparaison de l'amplitude du segment moyen du premier type par rapport à des données stockées en mémoire relatives à l'évolution des segments de ce premier type sur une période donnée et une comparaison de l'amplitude du segment moyen du troisième type de segment S3 par rapport à des données en mémoire relatives à l'évolution des segments de ce premier type sur une période donnée, qui peut être différente de la période sur laquelle les segments du premier type sont considérés.

Notamment, l'étape de détermination 114 peut résulter en une information de risque de décompensation cardiaque, lorsqu'il est constaté que le segment de premier type S1 du signal accélérométrique traité ACC_T est inférieur à ce qui a été enregistré précédemment, et que le segment de troisième type S3 du signal accélérométrique traité ACC_T est supérieur à ce qui a été enregistré précédemment.

L'analyse des sons cardiaques donne des informations sur la fonction hémodynamique. Dans le cas de l'insuffisance cardiaque, le premier son cardiaque, c'est-à-dire le segment de premier type S1, et le troisième son cardiaque, c'est-à-dire le segment de troisième type S3, sont tous deux intéressants pour la prédiction de la décompensation.

L'amplitude du segment de premier type S1 est fortement corrélée à la contractilité ventriculaire et aux performances cardiaques globales. Une diminution de son amplitude traduit une diminution de la contractilité cardiaque. A l'inverse, une augmentation de l'amplitude du segment de troisième type S3 est un signe spécifique d'une augmentation des pressions de remplissage consécutive à la diminution de la contractilité cardiaque.

Plusieurs études ont montré que la spécificité d'une augmentation de l'amplitude du segment de troisième type S3 dans les décompensations cardiaques est très élevée, de l'ordre de 80 %.

On va maintenant décrire, en référence à la figure 2, un premier mode de réalisation.

Tel qu'évoqué, l'étape de découpage 106 du signal accélérométrique ACC en cycles temporels 107 d'une durée déterminée tient notamment compte, dans ce mode de réalisation, d'un signal électrocardiogramme ECG. Il en résulte, préalablement à cette étape de découpage 106, une étape d'acquisition de ce signal électrocardiogramme ECG, réalisée simultanément à l'étape d'acquisition du signal accélérométrique dans une étape commune d'acquisition 102.

L'acquisition de ces deux signaux distincts est réalisée de manière synchrone, c'est-à-dire avec un déclenchement simultané des opérations d'acquisition, et avec une acquisition sur une même échelle de temps.

L'unité de commande est configurée pour réaliser en parallèle une première étape d'analyse 121 de signal au cours duquel le signal électrocardiogramme ECG est analysé et une deuxième étape d'analyse 122 de signal au cours duquel le signal accélérométrique ACC est analysé.

La première étape d'analyse 121 de signal comporte au moins une étape de filtration du signal électrocardiogramme 124 pour éliminer des artefacts et une étape de détection de pics du signal 126 représentatifs de caractéristiques cardiaques définies. L'unité de commande est configurée pour définir en sortie de cette première étape d'analyse 121 au moins une donnée sur la position temporelle de ces pics par rapport au début de la période d'acquisition.

La première étape d'analyse 121 de signal peut notamment comprendre, sans que cela soit limitatif de l'invention, au moins une filtration du signal électrocardiogramme ECG, par exemple sur une bande passante de l'ordre de 5-60 Hz, à la suite de laquelle est mise en œuvre une étape de détection de la positions de différents pics R qui se reproduisent successivement au cours de la période d'acquisition du signal, ces pics R étant représentatifs de caractéristiques cardiaques définies.

La position de ces pics, ici les pics R, est considérée en combinaison avec un signal accélérométrique filtré SCG obtenu lors d'une deuxième étape d'analyse de signal 122.

L'unité de commande est configurée pour réaliser, en parallèle de la première étape d'analyse de signal 121, la deuxième étape d'analyse de signal 122 au cours duquel le signal accélérométrique ACC acquis est analysé.

La deuxième étape d'analyse de signal 122 comporte au moins une étape de filtration du signal accélérométrique ACC. Plus particulièrement, le signal accélérométrique peut être filtré sur une bande passante comprise entre 5 et 100 Hz. Notamment, la fréquence peut être de l'ordre de 20 Hz pour s'affranchir du bruit basse fréquence et des fréquences liées au mouvement cardiaque.

L'unité de commande est configurée pour réaliser une étape d'analyse croisée du signal électrocardiogramme, et notamment de la position des pics R identifiés dans le signal électrocardiogramme filtré ECG_1 précédemment évoqué, avec le signal accélérométrique filtré SGC résultant de la deuxième étape d'analyse de signal 122.

L'étape d'analyse croisée consiste, dans une opération de synchronisation, à définir une origine de cycle temporel sur la base de l'apparition d'un pic R sur le signal électrocardiogramme filtré ECG_1 , et elle permet la réalisation de l'étape de découpage 106 du signal accélérométrique, ici filtré SGC, en une pluralité de cycles temporels 107 débutant respectivement à chaque pic R identifié et présentant une même durée.

On pourrait prévoir qu'une durée fixe, préalablement définie, est appliquée à chaque cycle temporel à partir de l'apparition d'un pic R. Dans l'exemple de réalisation de l'invention qui est ici décrit, chaque cycle temporel débutant par l'apparition d'un pic R se finit par l'apparition du pic R suivant, qui sert ainsi à définir la fin du cycle temporel n et le début du cycle temporel n+1. Afin de s'assurer que chaque cycle temporel présente la même durée, l'unité de commande est configurée pour ajuster la durée des cycles temporels en ajoutant du temps ou en retranchant par rapport à la durée moyenne de l'ensemble des cycles temporels ainsi identifiés par l'analyse croisée du signal accélérométrique et de la position des pics R sur le signal électrocardiogramme.

Un tel découpage d'un signal accélérométrique est notamment visible sur la figure 3, qui rend visible le découpage d'un signal accélérométrique en six cycles temporels 107 de même durée.

Une fois le signal accélérométrique filtré SCG segmenté en cycles temporels 107, l'étape d'identification 108 peut comporter une étape de calcul d'enveloppe pour chaque cycle temporel 107 au cours de laquelle on fait une approximation par enveloppe 109 du tracé d'une courbe représentative du signal cardiaque sur l'ensemble du cycle temporel, ce calcul pouvant tout aussi bien être un calcul d'enveloppe au carré qu'un calcul d'enveloppe en valeur absolue.

Un tel calcul d'enveloppe 109 pour chaque cycle temporel est notamment visible sur la figure 4, qui rend visible pour chaque cycle temporel une enveloppe au carré et une enveloppe en valeur absolue. On comprend que toute méthode permettant un redressement du signal pourrait ici être mise en œuvre sans sortir du contexte de l'invention.

L'étape d'identification 108 comporte par ailleurs une étape d'identification de segments S1, S2, ou S3 spécifiques du cycle temporel traité, en détectant les valeurs d'amplitude de l'une et ou l'autre des enveloppes précédemment calculées et qui s'écartent significativement d'un tracé plat du signal cardiaque. L'unité de commande identifie ainsi la présence d'un segment d'un premier type S1 lorsque l'amplitude du signal, ou plus particulièrement l'amplitude de l'une et/ou l'autre des enveloppes, est supérieure à une première valeur seuil. L'unité de commande identifie de la même façon la présence d'un segment d'un deuxième type S2 lorsque l'amplitude du signal, ou plus particulièrement l'amplitude de l'une et/ou l'autre des enveloppes, est supérieure à une deuxième valeur seuil, et la présence d'un segment d'un troisième type S3 lorsque l'amplitude du signal, ou plus particulièrement l'amplitude de l'une et/ou l'autre des enveloppes, est supérieure à une troisième valeur seuil.

Un segment est défini sur une plage temporelle donnée au cours de laquelle l'amplitude de l'enveloppe du signal accélérométrique est supérieure à la valeur seuil correspondante, étant entendu qu'une amplitude de l'enveloppe supérieure à la valeur seuil correspondante sur une durée trop courte peut être considérée comme un artefact et n'est pas assimilée comme un segment caractéristique.

Afin de s'assurer que les premiers, deuxièmes et troisièmes segments soient correctement identifiés, l'unité de commande réalise ces comparaisons des valeurs d'amplitude d'enveloppe avec une valeur seuil sur deux parties distinctes de chaque cycle temporel 107.

Plus particulièrement, l'unité de commande recherche des valeurs d'amplitude du signal supérieures à ladite première valeur seuil sur un premier tiers T1 du cycle temporel 107 .

En d'autres termes, les segments du premier type S1 sont recherchés et identifiés uniquement sur une première partie du cycle, ici un premier tiers T1. On entend par premier tiers T1 du cycle temporel 107 le laps de temps du cycle temporel s'écoulant depuis l'origine sur une durée égale à un tiers de la durée totale du cycle temporel.

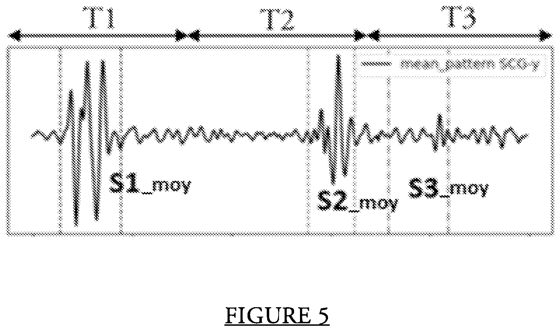

La figure 5, qui sera décrite ci-après et qui illustre un cycle temporel représentatif du signal accélérométrique traité, rend visible le découpage en trois tiers T1, T2, T3 du cycle temporel. Il convient de comprendre que dans cette étape d'identification, chaque cycle temporel est découpé de la sorte en trois tiers et que les segments des différents types sont recherchés dans le tiers qui lui correspond.

L'unité de commande est par ailleurs configurée pour rechercher des valeurs d'amplitude de l'enveloppe supérieures à des seuils de valeurs différentes de ladite première valeur seuil sur la suite du cycle temporel. En d'autres termes, les segments du deuxième type S2 et les segments du troisième type S3 sont recherchés et identifiés uniquement sur la partie du cycle temporel complémentaires à la première partie du cycle, c'est-à-dire ici les deux derniers tiers T2, T3 du cycle temporel 107.

Sont assimilés comme segments du deuxième type S2 les valeurs d'amplitude d'enveloppe supérieures à une deuxième valeur seuil et sont assimilés comme segments du troisième type S3 les valeurs d'amplitude d'enveloppe qui sont d'une part supérieures à une troisième valeur seuil, et qui sont apparues après les valeurs d'amplitude correspondant aux segments du deuxième type S2.

Il est notable dans cette étape d'identification que les segments du troisième type S3 sont recherchés à la suite des segments du deuxième type S2 détectés, dans une même partie du cycle temporel, à savoir la partie restante du cycle temporel dans laquelle les segments du premier type S1 n'ont pas été recherchés.

L'unité de commande est configurée pour mettre en œuvre une étape de tri 130 des segments identifiés, notamment pour éliminer au sein de ces segments ceux pouvant être considérés comme des artefacts, c'est-à-dire ici comme des segments trop distincts de ce qui est attendu pour le patient, via un calcul d'énergie spectrale tel qu'il a pu être présenté précédemment, c'est-à-dire en considérant un z-score de chaque segment.

En d'autres termes, cette étape de tri 130 consiste en une sous-étape d'exclusion de l'étape d'identification 108, qui consiste à exclure des segments préalablement identifiés comme étant des segments caractéristiques du son cardiaque identifiés, dès lors qu'ils sont classés lors de cette étape de tri comme étant des artefacts.

Les segments identifiés sont ici considérés comme des artefacts si le z-score qui leur est associé est supérieur à 3.

En sortie de cette étape d'identification 108, l'unité de commande est apte à stocker l'ensemble des segments de premier type S1 identifiés sur l'ensemble des cycles temporels 107 dans une première base de données, et l'ensemble des segments de deuxième type S2 et troisième type S3 identifiés sur l'ensemble des cycles temporels dans une deuxième base de données dans laquelle chaque segment de deuxième type est associé au segment de troisième type présent dans le même cycle temporel.

Il convient de noter que de manière alternative, dans un mode de réalisation qui va être décrit ci-après, l'unité de commande peut être configuré pour stocker les segments de troisième type S3 dans une troisième base de données, distinctement des segments de deuxième type S2.

Dans ce premier mode de réalisation, l'étape de sélection 110 de la cohérence des segments identifiés qui est mise en œuvre après l'étape d'identification 108 est scindée en deux sous-étapes menées en parallèle, au cours de chacune desquelles l'ensemble des segments d'un même type identifiés au préalable est traité.

Une première sous-étape 1101 consiste à intercorréler l'ensemble des segments du premier type identifiés au préalable pour sélectionner les segments du premier type cohérents qui vont être utilisés par la suite pour calculer un segment de premier type moyen.

Plus particulièrement, la première sous-étape 1101 comporte une étape d'intercorrélation 1101_1 de chacun des segments de premier type S1 pour définir un segment du premier type de référence S1_ref. En d'autres termes, chacun des segments de premier type S1 est corrélé avec l'ensemble des autres segments de premier type et une note de corrélation moyenne est attribuée à chaque cycle temporel sur la base des notes de corrélation établies en fonction de la cohérence du segment du premier type S1 associé à ce cycle temporel avec chacun des autres segments du premier type associés aux autres cycles temporels. Le segment de référence, et le cycle temporel de référence correspondant, est celui qui s'intercorrèle le mieux avec tous les autres.

On réalise ensuite, au cours de la première sous-étape 1101, une étape de tri par cohérence 1101_2, en comparant chaque segment de premier type S1 présent dans la base de données, et non éliminé par l'analyse d'énergie spectrale précédente, avec le segment de premier type de référence S1_ref de manière à définir un indice de corrélation. L'unité de commande est configurée pour ne pas retenir les segments de premier type pour lesquels l'indice de corrélation est inférieur à un seuil défini, qui peut être de l'ordre d'au moins 50% et ici de l'ordre de 60% sans que cela soit limitatif de l'invention.

Enfin, dans une étape de recalage 1101_3 de cette première sous-étape 1101, l'unité de commande est configurée pour recaler temporellement, sur le segment de premier type de référence S1_ref, chacun des segments de premier type S1 qui ont été conservés, c'est-à-dire chacun des segments qui n'ont pas été exclus de l'analyse par le calcul d'énergie spectrale ou par l'étape de tri par cohérence. Le recalage est réalisé de sorte à obtenir un maximum de corrélation entre les segments recalés et le segment de référence correspondant.

Sur la base de ces segments recalés et du segment de référence, le procédé met alors en œuvre l'étape de calcul 112 d'un segment moyen, ici l'étape de calcul 1121 d'un segment de premier type moyen S1_moy, représentatif de chacun des segments de premier type S1 identifiés et non exclus. Cette étape de calcul consiste à réaliser, pour chaque instant du cycle temporel et par exemple chaque milliseconde, un calcul de moyenne des valeurs d'amplitude de chacun des segments de premier type S1.

L'unité de commande est configurée pour stocker le segment de premier type moyen S1_moy associé à l'analyse du signal accélérométrique.

Simultanément à la réalisation de la première sous-étape 1101, l'unité de commande réalise une deuxième sous-étape 1102. Cette deuxième sous-étape 1102 comporte les mêmes étapes que la première sous-étape 1101, à savoir une étape de calcul d'intercorrélation 1102_1 pour définir un segment de deuxième type de référence S2_ref, une étape de tri par cohérence 1102_2 par rapport au segment de référence, une étape de recalage temporel 1102_3 et elle est également suivie par une étape de calcul 1122 d'un segment moyen. Là encore, cette deuxième sous-étape, et les différentes étapes qu'elle comporte, n'est effectuée que sur les segments ayant passés l'étape de l'analyse d'énergie spectrale, ce qui permet de concentrer les calculs uniquement sur les segments utiles.

Dans ce premier mode de réalisation, la deuxième sous-étape 1102 est réalisée en considérant uniquement les segments de deuxième type S2, les segments de troisième type S3 étant conservés ou écartés en fonction de ce qui est fait pour le segment de deuxième type S2 présent dans leur cycle temporel correspondant.

Plus particulièrement, le tri des segments, et donc des cycles temporels, par énergie spectrale est effectué en calculant le z-score des segments de deuxième type S2. Si un segment de deuxième type présente un z-score inférieur à une valeur seuil, ici égale à 0.3, l'ensemble du cycle temporel, c'est-à-dire le segment de deuxième type et le segment de troisième type associé, est exclu.

De manière similaire, l'étape de calcul d'intercorrélation 1102_1 est effectuée sur la base des segments de deuxième type, de sorte qu'un segment de deuxième type de référence S2_ref est choisi. Chacun des segments de deuxième type S2 stockés dans la deuxième base de données, et non exclus par l'analyse spectrale, est alors comparé au segment de deuxième type de référence S2_ref. Conformément à ce qui a été décrit pour la première sous-étape 1101, l'unité de commande sort de l'analyse les segments de deuxième type pour lesquels la comparaison avec le segment de deuxième type de référence résulte en un seuil de corrélation jugé insuffisant, qui à titre d'exemple ici est de l'ordre de 60% sans que cette valeur soit limitative. Toutefois, ici, cette exclusion des segments de deuxième type S2 a pour effet d'exclure également les segments de troisième type S3 identifiés dans un cycle temporel 107 correspondant à un segment de deuxième type S2 exclu.

L'étape de calcul de valeur moyenne des segments se fait alors pour chacun des types de segments, à savoir les segments de deuxième type S2 et les segments de troisième type S3.

L'unité de commande est configurée pour stocker le segment de deuxième type moyen S2_moy et le segment de troisième type moyen S3_moy, associés à l'analyse du signal accélérométrique. Ces segments moyens représentatifs du signal accélérométrique traité ACC-T servent par la suite à l'étape de détermination d'un risque de décompensation cardiaque 114, notamment en analysant l'évolution des amplitudes des segments d'un signal à l'autre.

L'unité de commande peut être configurée, à des fins de visualisation du signal, pour reconstituer un cycle temporel représentatif du signal accélérométrique traité ACC-T, sur la base du segment de premier type moyen S1_moy, du segment de deuxième type moyen S2_moy et du segment de troisième type moyen S3_moy.

La figure 5 illustre à titre d'exemple un cycle temporel 107 moyen, comportant un segment d'un premier type moyen S1_moy, présent dans le premier tiers T1 du cycle temporel, ainsi qu'un segment d'un deuxième type moyen S2_moy et un segment d'un troisième type S3_moy qui sont présents dans le reste du cycle temporel, à savoir dans le deuxième tiers T2 et T3.

On comprend que ce premier mode de réalisation est particulier en ce qu'il permet de reconstituer un signal avec un segment de troisième type moyen S3_moy, sans pour autant que les calculs d'intercorrélation soient réalisés sur ces types de segments mais sur des segments de deuxième type, d'amplitudes plus importantes et donc permettant un calcul d'intercorrélation et de cohérence plus fiable.

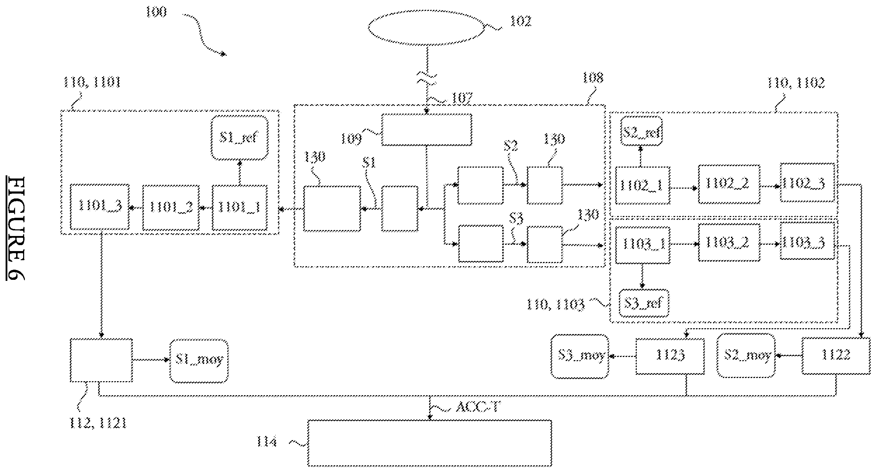

On va maintenant décrire, en référence à la figure 6, un deuxième mode de réalisation, dans lequel la deuxième sous-étape 1102 se distingue de la deuxième sous-étape du premier mode de réalisation en ce que les étapes d'intercorrélation pour déterminer un segment de référence et de tri par comparaison des segments au segment de référence sont réalisées aussi bien pour les segments de deuxième type que pour les segments de troisième type S3. Le segment de troisième type moyen S3_moy est calculé par l'unité de commande sur la base d'un tri des segments du troisième type identifiés qui est réalisé indépendamment du tri des segments du deuxième type identifiés.

Les étapes préalables à l'étape d'identification 108 sont ici sensiblement les mêmes de sorte qu'elles n'ont pas été représentées sur la figure 6.

Conformément à ce qui a été décrit précédemment, l'étape de sélection 110 de la cohérence des segments identifiés qui est mise en œuvre après l'étape d'identification 108 est scindée en plusieurs sous-étapes menées en parallèle, au cours de chacune desquelles l'ensemble des segments d'un même type identifiés au préalable est traité. Comme précédemment, une première sous-étape 1101 consiste à intercorréler l'ensemble des segments du premier type identifiés au préalable pour sélectionner les segments du premier type cohérents et pour calculer par la suite un segment de premier type moyen S1_moy.

Tel qu'évoqué précédemment, dans ce deuxième mode de réalisation, l'unité de commande est configurée pour stocker les segments de troisième type S3 dans une base de données distincte de la base de données dans laquelle sont stockés les segments de deuxième type S2.

Ainsi, dans ce deuxième mode de réalisation, l'étape de sélection 110 de la cohérence des segments est scindée en trois sous-étapes. Simultanément à la réalisation de la première sous-étape 1101, l'unité de commande réalise une deuxième sous-étape 1102 et une troisième sous-étape 1103. La deuxième sous-étape 1102 et la troisième sous-étape 1103 comportent respectivement les mêmes étapes que la première sous-étape 1101, à savoir une étape de calcul d'intercorrélation 1102_1, 1103_1 pour définir un segment de référence S2_ref, S3_ref, une étape de tri par cohérence 1102_2, 1103_2 par rapport au segment de référence et une étape de recalage temporel 1102_3, 1103_3 et chacune de ces deux sous-étapes est respectivement suivie d'une étape de calcul 1122, 1123 d'un segment moyen S2_moy, S3_moy.

On comprend ainsi que dans ce deuxième mode de réalisation, à la différence du premier mode de réalisation, un segment de troisième type de référence S3_ref est déterminé par un calcul d'intercorrélation spécifique aux segments de troisième type S3. La deuxième sous-étape 1102 est réalisée en considérant uniquement les segments de deuxième type S2 et la troisième sous-étape 1103 est réalisée en considérant uniquement les segments de troisième type S3, les segments étant conservés ou exclus dans chaque sous-étape uniquement en fonction de ce qui est fait au sein de la sous-étape correspondante.

A titre d'exemple, dans ce deuxième mode de réalisation, il est possible que le segment de troisième type S3 présent dans un cycle temporel défini 107 soit conservé car il est cohérent avec le segment de troisième type de référence S3_ref alors que simultanément le segment de deuxième type S2 présent dans ce même cycle temporel défini 107 est exclu car il est n'est pas cohérent avec le segment de deuxième type de référence S2_ref. L'étape de calcul 1122 de moyenne en fin de deuxième sous-étape 1102 peut être ainsi basée sur un nombre de segments du deuxième type S2 qui est différent du nombre de segments du troisième type S3 sur lequel est basée l'étape de calcul 1123 de moyenne en fin de troisième sous-étape 1103.

Là encore, un tri 130 des segments par analyse spectrale est réalisé, ici aussi bien pour les segments de premier type dans la première sous-étape 1101 et pour les segments de deuxième type S2 dans la deuxième sous-étape 1102 que pour les segments de troisième type S3 dans la troisième sous-étape 1103.

Ce tri présente le même intérêt que dans le premier mode de réalisation, à savoir éviter d'intégrer dans les calculs d'intercorrélation des segments qui sont manifestement atypiques et qui sont à considérer comme des artefacts. On réduit ainsi le temps de calcul associé à chaque sous-étape.

Conformément à ce qui a été évoqué pour le premier mode de réalisation, l'unité de commande est alors apte à stocker les segments moyens de chacun des types de segments et à les restituer pour l'étape de détermination d'un risque de décompensation cardiaque 114, au cours de laquelle on analyse notamment l'évolution des amplitudes des segments d'un signal à l'autre.

Là encore, il est possible de reconstituer, pour des fins de visualisation, un signal accélérométrique traité ACC-T sur la base du segment de premier type moyen S1_moy, du segment de deuxième type moyen S2_moy et du segment de troisième type moyen S3_moy.

On comprend que ce deuxième mode de réalisation est particulier en ce qu'il permet de reconstituer un signal avec un segment de troisième type moyen S3_moy, basé sur l'analyse directe de ces segments de troisième type.

L'invention telle qu'elle vient d'être décrite permet de répondre au but qu'elle s'était fixé, à savoir de traiter efficacement un signal accélérométrique acquis sur un patient pour en définir un signal traité permettant la détermination fiable d'une augmentation et ou d'une diminution de l'amplitude de certaines données au sein de ce signal, afin de donner au praticien des outils au diagnostic d'une décompensation cardiaque éventuelle. Des variantes non décrites ici pourraient être mises en œuvre, dès lors qu' elles s'inscrivent dans un procédé de détermination permettant notamment de définir des segments moyens d'au moins trois type représentatifs d'un signal accélérométrique acquis sur le patient.

A titre d'exemple non exhaustif, une variante possible est de prévoir une étape additionnelle dans laquelle on analyse le nombre de segments de chaque type qui sont conservés à la suite d'une étape du procédé et dans laquelle on compare ce nombre à une valeur seuil. Si le nombre de segments de l'un des types, par exemple le nombre de segments de premier type, est inférieur à ladite valeur seuil, le signal acquis est considéré comme étant de mauvaise qualité et n'est pas conservé pour calculer des segments moyens, de sorte que ce signal acquis n'est pas traité en vue de l'étape de détermination d'un risque de décompensation cardiaque. La valeur seuil peut par exemple être un nombre correspondant à 50% du nombre de cycles temporels résultant du découpage du signal accélérométrique. L'étape de procédé à la suite de laquelle cette étape additionnelle est mise en place peut notamment être l'étape d'identification des segments et la sous-étape d'exclusion avec calcul d'un score d'analyse spectrale. Les nombres de segments de premier type S1, de deuxième type S2 et de troisième type S3, qui sont respectivement conservés à la suite de cette sous-étape d'exclusion, sont comparés à ladite valeur seuil et une décision de poursuivre le procédé, et notamment d'engager l'étape de tri par cohérence, pour laquelle beaucoup de calculs d'intercorrélation sont à réaliser, est prise en fonction du résultat de cette comparaison. Comme évoqué ci-dessus, si un des nombres de segments d'un type donné est inférieur à la valeur seuil, l'acquisition du signal accélérométrique dans son ensemble est jugée peu fiable et aucun calcul n'est effectué par la suite sur la base de ce signal accélérométrique acquis.

Une autre variante possible serait de prévoir une autre sous-étape d'exclusion, qui aurait lieu au cours de l'étape de découpage en cycles temporels et qui consisterait à exclure du procédé les cycles temporels dont la durée, notamment définie par l'intervalle entre deux pics R successifs, est beaucoup plus longue que la valeur moyenne, ou la valeur médiane, des durées des autres cycles temporels. A titre d'exemple, les cycles temporels ayant une durée à l'origine supérieure à 1,7 fois ladite valeur moyenne ou médiane sont exclus du reste du procédé et ne sont ainsi ni recalés sur la durée moyenne des cycles temporels ni analysés pour détecter des segments caractéristiques. De manière analogue, les cycles temporels ayant une durée trop courte, par exemple ayant une durée à l'origine inférieure à 0,3 fois ladite valeur moyenne ou médiane, sont également exclus.

## Revendications

1. Unité de commande pour la détermination d'un risque de décompensation cardiaque d'un patient, l'unité de commande étant configurée pour traiter un signal accélérométrique (ACC) acquis sur le patient pour la détermination d'un risque de décompensation cardiaque en se basant sur l'étude d'une pluralité de marqueurs (115, 117, 119, PEP) parmi lesquels des segments (S1, S2, S3) d'un signal accélérométrique traité (ACC-T) résultant du traitement par l'unité de commande,
l'unité de commande comportant :
- des moyens de découpage du signal accélérométrique en cycles temporels (107) d'une même durée ;
- des moyens d'identification , dans chaque cycle temporel (107), de segments (S1, S2, S3) caractéristiques du son cardiaque, configurés pour mettre en œuvre une première recherche d'un premier type de segment (S1) dans une première partie définie du cycle temporel (107) et une deuxième recherche d'au moins un deuxième type de segment (S2) et un troisième type de segment (S3) dans une deuxième partie définie du cycle temporel (107),
- des moyens de sélection de la cohérence des segments identifiés, au moins pour les premiers types de segments (S1) et les deuxièmes types de segments (S2), les moyens de sélection étant configurés pour intercorréler l'ensemble des segments du même type identifiés au préalable pour pouvoir en déduire un segment de référence pour ce type de segments et pour pouvoir dans un deuxième temps ne garder que les segments se rapprochant au plus près de ce segment de référence, c'est-à-dire les segments cohérents de chacun des types de segment,
- des moyens de calcul d'un segment d'un premier type moyen (S1 moy), d'un segment d'un deuxième type moyen (S2_moy) et d'un segment d'un troisième type moyen (S3_moy), le calcul de moyenne étant basé sur tous les segments d'un premier type (S1) sélectionnés, respectivement tous les segments d'un deuxième type (S2) sélectionnés, respectivement tous les segments d'un troisième type (S3) sélectionnés,
la détermination du risque de décompensation cardiaque comportant la détermination d'une augmentation de l'amplitude du premier type de segment (S1) par rapport à des données en mémoire et/ou d'une diminution de l'amplitude du troisième type de segment (S3) par rapport à des données en mémoire, l'unité de commande étant configurée pour d'une part stocker le segment de premier type moyen (S1_moy) associé à l'analyse du signal accélérométrique et pour d'autre part stocker le segment de deuxième type moyen (S2_moy) et le segment de troisième type moyen (S3_moy), associés à l'analyse du signal accélérométrique, ces segments moyens représentatifs du signal accélérométrique traité (ACC-T) servant à la détermination, au cours de laquelle on analyse notamment l'évolution des amplitudes des segments d'un signal à l'autre.

2. Unité de commande selon la revendication 1, dans laquelle les moyens d'identification sont configurés pour mettre en œuvre une exclusion de segments préalablement identifiés comme étant des segments de premier, deuxième ou troisième types, et classés lors de cette sous-étape d'exclusion comme étant des artefacts.

3. Unité de commande selon la revendication précédente, dans laquelle les moyens d'identification configurés pour mettre en œuvre l'exclusion sont configurés pour mettre en œuvre un calcul d'un score d'analyse spectrale, les segments présentant un score supérieur à une valeur seuil étant identifiés comme étant des artefacts et exclus.

4. Unité de commande selon la revendication 1, configurée pour traiter chacun des signaux détectés sur l'un des trois axes d'acquisition du signal accélérométrique (ACC) ou bien un signal d'ensemble résultant de la combinaison des signaux acquis sur chacun des axes.

5. Unité de commande selon la revendication 1, configurée pour traiter un signal électrocardiogramme (ECG) acquis sur le patient.

6. Unité de commande selon la revendication précédente, **caractérisée en ce que** les moyens de découpage du signal sont configurés pour mettre en œuvre une opération de synchronisation du signal accélérométrique (ACC) sur la base du signal électrocardiogramme (ECG).

7. Unité de commande selon l'une des revendications précédentes, **caractérisée en ce que** les moyens d'identification sont configurés pour mettre en œuvre un calcul d'enveloppe (109)du signal accélérométrique, un segment (S1, S2, S3) étant identifié lorsque l'amplitude de l'enveloppe (109) est supérieure à un seuil déterminé dans une partie définie du cycle temporel (107).

8. Unité de commande selon l'une des revendications précédentes, **caractérisée en ce que** la première partie définie du cycle temporel (107) par les moyens d'identification est égale au premier tiers (T1) de la durée dudit cycle temporel (107), la deuxième partie définie du cycle temporel étant égale au deux derniers tiers (T2, T3) dudit cycle temporel (107).

9. Unité de commande selon l'une des revendications précédentes, **caractérisée en ce que** les moyens de sélection sont configurés pour mettre en œuvre au moins deux suites de calculs de corrélation effectuées en parallèle, parmi lesquelles une première suite de calculs de corrélation réalisée sur l'ensemble des segments du premier type (S1) et une deuxième suite de calculs de corrélation réalisée sur l'ensemble des segments du deuxième type (S2).

10. Unité de commande selon la revendication précédente, **caractérisée en ce que** les moyens de sélection sont configurés pour mettre en œuvre, pour chaque suite de calculs de corrélation effectué pour un type de segment donné (S1, S2, S3),un premier calcul d'intercorrélation (1101_1 ; 1102_1 ; 1103_1) entre l'ensemble des segments identifiés comme étant des segments de ce type, pour définir un segment de référence (S1_ref ; S2_ref ; S3_ref) pour ce type de segment, les moyens de sélection étant configurés pour mettre en œuvre un calcul de corrélation de chacun des segments identifiés comme étant des segments de ce type par rapport au segment de référence.

11. Unité de commande selon la revendication précédente, **caractérisée en ce que** les moyens de sélection sélectionnent dans chaque suite de calculs de corrélation les segments qui ont un indice de corrélation avec le segment de référence (S 1 _ref ; S2_ref ; S3_ref) qui est supérieur ou égal à un seuil de corrélation prédéterminé.

12. Unité de commande selon l'une des revendications 9 à 11, **caractérisée en ce que** les moyens de sélection sont configurés pour mettre en œuvre uniquement deux suites de calculs de corrélation effectuées en parallèle, parmi lesquelles une première suite de calcul de corrélation réalisée sur l'ensemble des segments du premier type (S1) et une deuxième suite de calcul de corrélation réalisée sur l'ensemble des segments du deuxième type (S2).

13. Unité de commande selon l'une des revendications 9 à 11, **caractérisée en ce que** les moyens de sélection sont configurés pour mettre en œuvre trois suites de calculs de corrélation effectuées en parallèle, parmi lesquelles une première suite de calcul de corrélation réalisée sur l'ensemble des segments du premier type (S1), une deuxième suite de calcul de corrélation réalisée sur l'ensemble des segments du deuxième type (S2), et une troisième suite de calcul de corrélation réalisée sur l'ensemble des segments du troisième type (S3).

14. Unité de commande selon l'une des revendications précédentes, en combinaison avec la revendication 10, **caractérisée en ce que** les moyens de sélection sont configurés pour effectuer, avant la mise en œuvre des moyens de calcul des segments moyens, un recalage temporel (1101_3 ; 1102_3 ; 1103_3) des segments sélectionnés sur le segment de référence (S1_ref ; S2_ref ; S3_ref) correspondant, pour chaque type de segments.

## Patentansprüche

1. Steuereinheit zum Bestimmen eines Risikos einer Herzdekompensation eines Patienten, wobei die Steuereinheit konfiguriert ist, um ein an dem Patienten erfasstes Beschleunigungssignal (ACC) zum Bestimmen eines Risikos einer Herzdekompensation basierend auf der Untersuchung einer Vielzahl von Markern (115, 117, 119, PEP) zu verarbeiten, darunter Segmente (S1, S2, S3) eines verarbeiteten Beschleunigungssignals (ACC-T), das aus der Verarbeitung durch die Steuereinheit resultiert,
die Steuereinheit umfassend:
- Mittel zum Unterteilen des Beschleunigungssignals in Zeitzyklen (107) gleicher Dauer;
- Mittel zum Identifizieren von für den Herzton charakteristischen Segmenten (S1, S2, S3) in jedem Zeitzyklus (107), die konfiguriert sind, um eine erste Suche nach einem ersten Segmenttyp (S1) in einem ersten definierten Teil des Zeitzyklus (107) und eine zweite Suche nach mindestens einem zweiten Segmenttyp (S2) und einem dritten Segmenttyp (S3) in einem zweiten definierten Teil des Zeitzyklus (107) durchzuführen,
- Mittel zum Auswählen der Kohärenz der identifizierten Segmente, mindestens für die ersten Segmenttypen (S1) und die zweiten Segmenttypen (S2), wobei die Auswahlmittel konfiguriert sind, um die Gesamtheit der zuvor identifizierten Segmente desselben Typs untereinander zu korrelieren, um daraus ein Referenzsegment für diesen Segmenttyp ableiten zu können und um in einem zweiten Schritt nur die Segmente behalten zu können, die diesem Referenzsegment am nächsten kommen, das heißt die kohärenten Segmente jedes der Segmenttypen,
- Mittel zum Berechnen eines mittleren Segments eines ersten Typs (S1_mittel), eines mittleren Segments eines zweiten Typs (S2_mittel) und eines mittleren Segments eines dritten Typs (S3_mittel), wobei die Mittelwertberechnung auf allen ausgewählten Segmenten eines ersten Typs (S1) bzw. allen ausgewählten Segmenten eines zweiten Typs (S2) bzw. allen ausgewählten Segmenten eines dritten Typs (S3) basiert,
wobei die Bestimmung des Risikos einer Herzdekompensation das Bestimmen einer Zunahme der Amplitude des ersten Segmenttyps (S1) relativ zu Daten in dem Speicher und/oder einer Abnahme der Amplitude des dritten Segmenttyps (S3) relativ zu Daten in dem Speicher umfasst, wobei die Steuereinheit konfiguriert ist, um einerseits das der Analyse des Beschleunigungssignals zugeordnete mittlere Segment des ersten Typs (S1_mittel) zu speichern und um andererseits das mittlere Segment des zweiten Typs (S2_mittel) und das mittlere Segment des dritten Typs (S3_mittel) zu speichern, die der Analyse des Beschleunigungssignals zugeordnet sind, wobei diese für das verarbeitete Beschleunigungssignal (ACC-T) repräsentativen mittleren Segmente für die Bestimmung dienen, in deren Verlauf insbesondere die Entwicklung der Amplituden der Segmente von einem Signal zum anderen analysiert wird.

2. Steuereinheit nach Anspruch 1, wobei die Identifizierungsmittel konfiguriert sind, um einen Ausschluss von Segmenten durchzuführen, die zuvor als Segmente eines ersten, zweiten oder dritten Typs identifiziert wurden und während dieses Teilschritts des Ausschlusses als Artefakte klassifiziert werden.

3. Steuereinheit nach dem vorstehenden Anspruch, wobei die Identifikationsmittel, die konfiguriert sind, um den Ausschluss zu implementieren, konfiguriert sind, um eine Berechnung eines Spektralanalyse-Scores durchzuführen, wobei die Segmente, die einen Score über einem Schwellenwert aufweisen, als Artefakte identifiziert und ausgeschlossen werden.

4. Steuereinheit nach Anspruch 1, konfiguriert, um jedes der auf einer der drei Erfassungsachsen des Beschleunigungssignals (ACC) erfassten Signale oder ein Gesamtsignal, das aus der Kombination der auf jeder der Achsen erfassten Signale resultiert, zu verarbeiten.

5. Steuereinheit nach Anspruch 1, konfiguriert, um ein an dem Patienten erfasstes Elektrokardiogrammsignal (EKG-Signal) zu verarbeiten.

6. Steuereinheit nach dem vorstehenden Anspruch,
**dadurch gekennzeichnet, dass** die Signalunterteilungsmittel konfiguriert sind, um einen Synchronisationsvorgang des Beschleunigungssignals (ACC) basierend auf dem Elektrokardiogrammsignal (EKG-Signal) durchzuführen.

7. Steuereinheit nach einem der vorstehenden Ansprüche,
**dadurch gekennzeichnet, dass** die Identifikationsmittel konfiguriert sind, um eine Berechnung der Hüllkurve (109) des Beschleunigungssignals durchzuführen, wobei ein Segment (S1, S2, S3) identifiziert wird, wenn die Amplitude der Hüllkurve (109) in einem definierten Teil des Zeitzyklus (107) größer als ein bestimmter Schwellenwert ist.

8. Steuereinheit nach einem der vorstehenden Ansprüche,
**dadurch gekennzeichnet, dass** der durch die Identifikationsmittel definierte erste Teil des Zeitzyklus (107) gleich dem ersten Drittel (T1) der Dauer des Zeitzyklus (107) ist, wobei der definierte zweite Teil des Zeitzyklus gleich den letzten beiden Dritteln (T2, T3) des Zeitzyklus (107) ist.

9. Steuereinheit nach einem der vorstehenden Ansprüche,
**dadurch gekennzeichnet, dass** die Auswahlmittel konfiguriert sind, um mindestens zwei parallel durchgeführte Folgen von Korrelationsberechnungen durchzuführen, darunter eine erste Folge von Korrelationsberechnungen, die an der Gesamtheit der Segmente des ersten Typs (S1) durchgeführt wird, und eine zweite Folge von Korrelationsberechnungen, die an der Gesamtheit der Segmente des zweiten Typs (S2) durchgeführt wird.

10. Steuereinheit nach dem vorstehenden Anspruch,
**dadurch gekennzeichnet, dass** die Auswahlmittel konfiguriert sind, um für jede Folge von Korrelationsberechnungen, die für einen gegebenen Segmenttyp (S1, S2, S3) durchgeführt wird, eine erste Kreuzkorrelationsberechnung (1101_1; 1102_1; 1103_1) zwischen der Gesamtheit der als Segmente dieses Typs identifizierten Segmente durchzuführen, um ein Referenzsegment (S1_ref; S2_ref; S3_ref) für diesen Segmenttyp zu definieren, wobei die Auswahlmittel konfiguriert sind, um eine Korrelationsberechnung jedes der als Segmente dieses Typs identifizierten Segmente relativ zu dem Referenzsegment durchzuführen.

11. Steuereinheit nach dem vorstehenden Anspruch,
**dadurch gekennzeichnet, dass** die Auswahlmittel in jeder Folge von Korrelationsberechnungen die Segmente auswählen, die einen Korrelationsindex mit dem Referenzsegment (S1_ref; S2_ref; S3_ref) aufweisen, der größer oder gleich einem vorbestimmten Korrelationsschwellenwert ist.

12. Steuereinheit nach einem der Ansprüche 9 bis 11,
**dadurch gekennzeichnet, dass** die Auswahlmittel konfiguriert sind, um nur zwei parallel durchgeführte Folgen von Korrelationsberechnungen durchzuführen, darunter eine erste Folge von Korrelationsberechnungen, die an der Gesamtheit der Segmente des ersten Typs (S1) durchgeführt wird, und eine zweite Folge von Korrelationsberechnungen, die an der Gesamtheit der Segmente des zweiten Typs (S2) durchgeführt wird.

13. Steuereinheit nach einem der Ansprüche 9 bis 11,
**dadurch gekennzeichnet, dass** die Auswahlmittel konfiguriert sind, um drei parallel durchgeführte Folgen von Korrelationsberechnungen durchzuführen, darunter eine erste Folge von Korrelationsberechnungen, die an der Gesamtheit der Segmente des ersten Typs (S1) durchgeführt wird, eine zweite Folge von Korrelationsberechnungen, die an der Gesamtheit der Segmente des zweiten Typs (S2) durchgeführt wird, und eine dritte Folge von Korrelationsberechnungen, die an der Gesamtheit der Segmente des dritten Typs (S3) durchgeführt wird.

14. Steuereinheit nach einem der vorstehenden Ansprüche in Kombination mit Anspruch 10, **dadurch gekennzeichnet, dass** die Auswahlmittel konfiguriert sind, um vor der Implementierung der Mittel zum Berechnen der mittleren Segmente eine zeitliche Ausrichtung (1101_3; 1102_3; 1103_3) der ausgewählten Segmente auf das entsprechende Referenzsegment (S1_ref; S2_ref; S3_ref) für jeden Segmenttyp durchzuführen.

## Claims

1. Control unit for determining the risk of cardiac decompensation in a patient, the control unit being configured to process an accelerometric signal (ACC) acquired from the patient for the determination of the risk of cardiac decompensation based on the study of a plurality of markers (115, 117, 119, PEP) including segments (S1, S2, S3) of a processed accelerometric signal (ACC-T) resulting from the processing by the control unit,
the control unit comprising:
- means of cutting the accelerometric signal into time cycles (107) of the same duration;
- means of identifying, in each time cycle (107), segments (S1, S2, S3) characteristic of the heart sound, configured to implement a first search for a first type of segment (S1) in a first defined part of the time cycle (107) and a second search for at least a second type of segment (S2) and a third type of segment (S3) in a second defined part of the time cycle (107),
- means for selecting the consistency of the identified segments, at least for the first segment types (S1) and the second segment types (S2), the selection means being configured for intercorrelate all previously identified segments of the same type in order to deduce a reference segment for this type of segment and then, in a second step, keep only the segments that most closely resemble this reference segment, i.e., the coherent segments of each segment type,
- means of calculating an average segment of a first type (S1_moy), an average segment of a second type (S2_moy) and an average segment of a third type (S3_moy), the average calculation being based on all selected segments of a first type (S1), respectively all selected segments of a second type (S2), respectively all selected segments of a third type (S3),
the determination of the risk of cardiac decompensation including the determination of an increase in the amplitude of the first type of segment (S1) compared to data in memory and/or a decrease in the amplitude of the third type of segment (S3) compared to data in memory, the control unit being configured to store on the one hand the average first type segment (S1_moy) associated with the analysis of the accelerometric signal and on the other hand to store the average second type segment (S2_moy) and the average third type segment (S3_moy), associated with the analysis of the accelerometric signal, these average segments representing the processed accelerometric signal (ACC-T) being used for the determination, during which the evolution of the amplitudes of the segments from one signal to another is analyzed.

2. Control unit according to claim 1, wherein the identification means are configured to implement an exclusion of segments previously identified as being segments of first, second or third type, and classified during this exclusion substep as being artifacts.

3. Control unit according to the preceding claim, wherein the identification means configured to implement the exclusion are configured to implement a calculation of a spectral analysis score, segments exhibiting a score greater than a threshold value being identified as artifacts and excluded.

4. Control unit according to claim 1, configured to process each of the signals detected on one of the three axes of acquisition of the accelerometer signal (ACC) or an global signal resulting from the combination of the signals acquired on each of the axes.

5. Control unit according to claim 1, configured to process an electrocardiogram (ECG) signal acquired from the patient.

6. Control unit according to the preceding claim, **characterized in that** the signal cutting means are configured to implement an accelerometer signal synchronization (ACC) operation based on the electrocardiogram (ECG) signal.

7. Control unit according to any one of the preceding claims, **characterized in that** the identification means are configured to implement a calculation of an envelope (109) of the accelerometer signal, a segment (S1, S2, S3) being identified when the amplitude of the envelope (109) is greater than a threshold determined in a defined part of the time cycle (107).

8. Control unit according to one of the preceding claims, **characterized in that** the first defined part of the time cycle (107) by the identification means is equal to the first third (T1) of the duration of said time cycle (107), the second defined part of the time cycle being equal to the last two thirds (T2, T3) of said time cycle (107).

9. Control unit according to one of the preceding claims, **characterized in that** the selection means are configured to implement at least two parallel correlation calculation suites, comprising a first correlation calculation suite carried out on all segments of the first type (S1) and a second correlation calculation suite carried out on all segments of the second type (S2).

10. Control unit according to the preceding claim, **characterized in that** the selection means are configured to implement, for each suites of correlation calculations performed for a given segment type (S1, S2, S3), a first cross-correlation calculation (1101_1; 1102_1; 1103_1) between all segments identified as being of that type, to define a reference segment (S1_ref; S2_ref; S3_ref) for that segment type, the selection means being configured to implement a correlation calculation of each of the segments identified as being segments of this type with respect to the reference segment.

11. Control unit according to the preceding claim, **characterized in that** the selection means select in each series of correlation calculations the segments which have a correlation index with the reference segment (S1_ref; S2_ref; S3_ref) which is greater than or equal to a predetermined correlation threshold.

12. Control unit according to any one of claims 9 to 11, **characterized in that** the selection means are configured to implement only two parallel correlation calculation suites, comprising a first correlation calculation suite carried out on all segments of the first type (S1) and a second correlation calculation suite carried out on all segments of the second type (S2).

13. Control unit according to any one of claims 9 to 11, **characterized in that** the selection means are configured to implement three parallel correlation calculation suites, comprising a first correlation calculation suite carried out on all segments of the first type (S1), a second correlation calculation suite carried out on all segments of the second type (S2), and a third correlation calculation suite carried out on all segments of the third type (S3).

14. Control unit according to one of the preceding claims, in combination with claim 10, **characterized in that** the selection means are configured to perform, before the implementation of the means for calculating the average segments, a time recalibration (1101_3; 1102_3; 1103_3) of the selected segments on the corresponding reference segment (S1_ref; S2_ref; S3_ref), for each type of segment.
